# EUROPEAN PATENT APPLICATION

(11) **EP 2 604 253 A1**
(43) Date of publication of application: **19.06.2013**
(21) Application number: 11193358.6
(22) Date of filing: 13.12.2011
(51) Int. Cl.: A61K 8/37, A61K 8/73, A61K 8/92, A61Q 19/00, A61K 8/02, A61K 8/06

(54) **Water-in-oil emulsions and methods for their preparation**

(71) Applicant: Glatter, Otto, 8042 Graz (AT)
(72) Inventor: Glatter, Otto, 8042 Graz (AT); Glatter, Ingo, 8010 Graz (AT)
(74) Representative: Danner, Stefan

(57) **Abstract**

The present invention relates to a water-in-oil emulsion, comprising a continuous hydrophobic phase in which the hydrophilic phase is dispersed, wherein: (i) the emulsion has a total water content in the range of 30% to 95% (w/w); (ii) the emulsion does not comprise an emulsion stabilizer; (iii) the hydrophobic phase has a ratio of lipids to oils in the range of 50% to 96% (w/w); and (iv) the continuous hydrophobic phase is made of lyotropic liquid crystalline nanostructures including 5% to 40% (w/w) water. Furthermore, the present invention relates to a corresponding method for preparing such water-in-oil emulsion, the method comprising: (a) pre-mixing of the hydrophilic phase and the hydrophobic phase at a rotation speed of at least 4.000 rpm and at a temperature of at least 40°C, thus forming a raw emulsion; (b) positioning the raw emulsion in a shear device; and (c) applying a shear rate of at least 4.500 s⁻¹; and cooling the emulsion to ambient temperature, thus forming a continuous hydrophobic phase of the emulsion that is made of lyotropic liquid crystalline nanostructures.

## Description

### FIELD OF THE INVENTION

The present invention relates to water-in-oil emulsions, and in particular to water-in-oil emulsions whose continuous hydrophobic phase is made of lyotropic liquid crystalline nanostructures without the use of stabilizing agents for the water-oil-interphase. The present invention further concerns corresponding methods for preparing such emulsions.

### BACKGROUND

In particular, for cosmetic and pharmaceutical formulations, it is of critical importance to have the active ingredients arranged in such a way that their functionality and bioavailability are optimized. Self-assembly of molecular structures is a central mechanism for efficient structural design (Robinson, B.H. (ed) (2003) Self-Assembly, IOS Press, Amsterdam). Moreover, for the successful incorporation of different functional molecules in such formulations, it is also necessary to generate systems with hydrophilic and hydrophobic regions with large interfacial area. For topical applications, emulsions are of particular interest. An emulsion is broadly-divided into oil-in-water (O/W) type and water-in-oil (W/O) types. In addition to these types, there are multi-type emulsions such as oil/water/oil (O/W/O) type and water/oil/water (W/O/W) type.

More recently, emulsion particles containing inverted-type liquid crystalline phases or inverse micellar solutions or water-in-oil microemulsions have attracted much attention due to their great potential for applications in various technical fields. For instance, the formulation of such nanostructured systems is attractive due to the possibility of combining the solubilization capacity of amphiphilic, hydrophobic, and hydrophilic drugs with their controlled release. For optimal and effective use, it is necessary to fragment the highly viscous mesophases (e.g., bicontinuous or micellar cubic and hexagonal liquid crystalline systems) into syringeable dispersions with low viscosity (reviewed in, e.g., Shah, J.C. et al. (2001) Adv. Drug Delivery Rev. 47, 229-250). In addition, these aqueous dispersions have to fulfill certain criteria concerning particle size and particle size distribution to avoid blocking the fine blood vessels or causing other serious side effects. Such internally nanostructured particles are commonly referred to as "Isasomes".

These kinetically stabilized particles have several advantages as compared to other conventional dispersions such as regular oil-in-water (O/W) emulsions or double emulsions (W/O/W) or other liposomal formulations which have been utilized for several decades for the delivery of different active molecules. This is due to their high internal interfacial area and their ability to solubilize hydrophilic, hydrophobic, as well as amphiphilic molecules. The internal phase typically contains a lipophilic amphiphile (e.g., monoglycerides or phytantriol), water, and oil (Yaghmur, A. et al. (2006) Langmuir22, 9919-9927).

So far, it has been possible to emulsify various liquid crystalline phases (reviewed in, e.g., Yaghmur, A. and Glatter, O. (2009) Adv. Colloid Interface Sci. 147-148, 333-342). However, the formation of stable dispersions with internal nanostructures could only be achieved by using an efficient stabilizer (for example, surfactants such as Pluronic® F127 or nanoparticles for "pickering-emulsions" [Salonen, A. et al. (2008) Langmuir 24, 5306-5314; Salonen, A. et al. (2010) Langmuir 26, 7981-7987]). However, the presence and amount of such stabilizers has been shown to have a strong influence on the internal nanostructures formed (Nakano, M. et al. (2002) Langmuir 18, 9283-9288). For example, high concentrations of surfactant may lead to a reduction of the particle size. Hence, the presence of such stabilizers poses constraints with regard to the physicochemical properties of the formulations that can be prepared and that may interfere with some intended applications.

For cosmetic formulations, a water-in-oil type emulsion is particularly appropriate because it can be efficiently spread on the skin and typically has a superior moisturizing behavior. In this regard, a water-in-oil type emulsion having a high water content is superior to an oil-in-water type emulsion. On the other hand, it is generally thought that keeping a water-in-oil type emulsion stable is more difficult compared with an oil-in-water type emulsion. In a water-in-oil type emulsion, stabilization was often improved by using mixtures of different ionic and non-ionic surfactants or a large amount of surfactant so that the viscosity of the oil constituting the outer phase was increased and droplets were immobilized. Thus, there has been a limitation in amounts and kinds of emulsifier, aqueous component, oil component, and other stabilizers as well as an inner water phase ratio.

It is also of note that equilibrium systems such as micelles or microemulsions have another important drawback: they may change dramatically, or even disassemble, if they are mixed with other systems or if just some parameters such as temperature or salinity are changed.

Therefore, there still remains a need for emulsions that overcome the above limitations. In particular, there is a need for stable water-in-oil emulsions that can be produced without addition of emulsifying or stabilizing agents, that have a highly ordered structure of the oil phase, and that enable the effective solubilization of active ingredients along with their controlled release.

Hence, it is an object of the present invention to provide such emulsions as well as corresponding methods for their preparation.

### SUMMARY OF THE INVENTION

In a first aspect, the present invention relates to a water-in-oil emulsion, comprising a continuous hydrophobic phase in which the hydrophilic phase is dispersed, wherein:
(i) the emulsion has a total water content in a range of 30% to 95% (w/w);
(ii) the emulsion does not comprise an emulsion stabilizer;
(iii) the hydrophobic phase has a ratio of lipids to oils in the range of 50% to 96% (w/w);
   and
(iv) the continuous hydrophobic phase is made of lyotropic liquid crystalline nanostructures including 5% to 40% (w/w) water.

In preferred embodiments, the lyotropic liquid crystalline nanostructures of the hydrophobic phase have a crystalline phase being selected from the group consisting of fluid isotropic, hexagonal, bicontinuous cubic, and discrete micellar cubic, with the latter three being particularly preferred.

In specific embodiments, the hydrophilic phase is dispersed in the continuous hydrophobic phase in form of droplets having a diameter in the range of 0.1 to 50 µm.

In further preferred embodiments, the total water content of the emulsion is at least 70% (w/w), and particularly at least 75% (w/w).

In particularly preferred embodiments, the oils employed in the hydrophobic phase are natural oils.

In further preferred embodiments, the hydrophilic phase of the emulsion further comprises a hydrogelator.

In a further aspect, the present invention relates to a method for preparing a water-in-oil emulsion comprising a continuous hydrophobic phase made of lyotropic liquid crystalline nanostructures as defined herein, the method comprising:
(a) pre-mixing of the hydrophilic phase and the hydrophobic phase at a rotation speed of at least 4.000 rpm and at a temperature of at least 40°C, thus forming a raw emulsion;
(b) positioning the raw emulsion in a shear device; and
(c) applying a shear rate of at least 4.500 s⁻¹, and cooling the emulsion to ambient temperature, thus forming a continuous hydrophobic phase of the emulsion that is made of lyotropic liquid crystalline nanostructures.

In preferred embodiments, the shear device employed is a Couette laminar flow shear device.

In particular embodiments, the method further comprises controlling the crystalline phase of the lyotropic liquid crystalline nanostructures formed in the hydrophobic phase by adjusting any one or more of the preparation parameters selected from the group consisting of preparation temperature, total water content of the emulsion, ratio of lipids to oils in the hydrophobic phase of the emulsion, and presence of a hydrogelator in the hydrophilic phase.

In specific embodiments, the pre-mixing step is performed at a temperature of at least 60°C.

In further preferred embodiments, the pre-mixing step of the method is performed at a rotation speed of at least 15.000 rpm, and a shear rate of at least 20.000 s⁻¹ is applied.

In another aspect, the present invention relates to the use of a water-in-oil emulsion as defined herein for the preparation of a cosmetic or pharmaceutical composition, the composition comprising one or more active ingredients.

In preferred embodiments, the cosmetic or pharmaceutical composition prepared is for topical application, in particular being selected from the group consisting of creams, lotions, mousses, and balms.

Other embodiments of the present invention will become apparent from the detailed description hereinafter.

### DESCRIPTION OF THE DRAWINGS

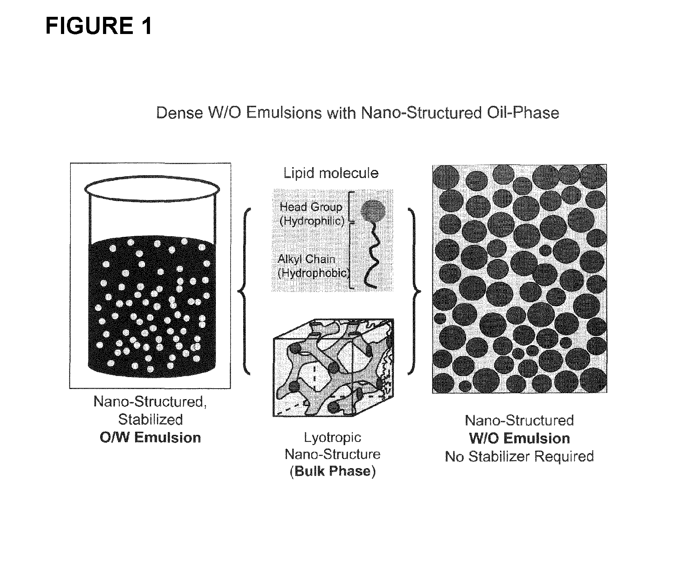

**FIGURE 1****: Schematic illustration of the basic principle underlying the invention.** On the left panel, a conventional oil-in-water dispersion comprising nanostructures is shown. A stabilizer is required to prepare and to stabilize the formulation. In contrast, for preparing a water-in-oil emulsion according to the invention, no emulsion stabilizer for the water-oil-interface is required (neither molecular nor colloidal). Stability of the formulation is rather accomplished by the formation of lyotropic liquid crystalline nanostructures and the dense packing of the dispersed hydrophilic phase.

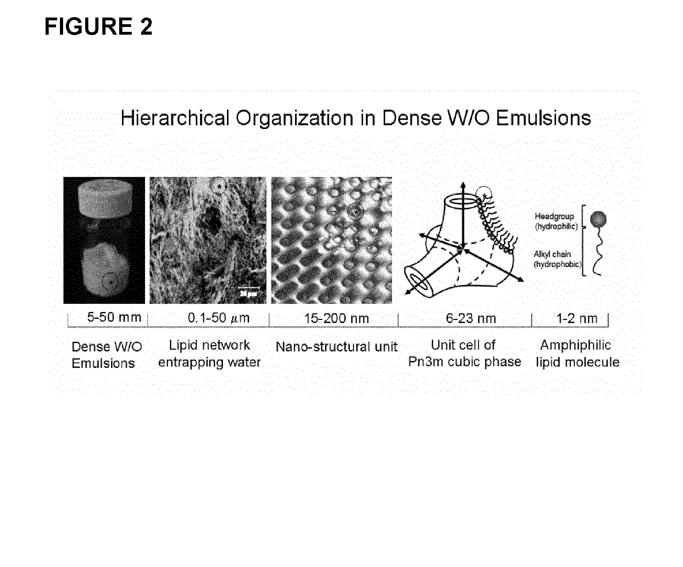

**FIGURE 2****: Hierarchical order in water-in-oil emulsions according to the invention.** On the left panel, a dense emulsion according to the invention is shown. The emulsion is further composed of varying levels of structural hierarchy (shown from right to left). The amphiphilic molecule (lipid or lipid + oil), consisting of hydrophilic head and hydrophobic tail, represents the basis of this structural ladder. Many such molecules then generate the next hierarchical level that further becomes an integral part of upper levels as represented by subsequent images. The encircled area compares the subsequent levels of structural order. The length scales were as follows: 1-2 nm: molecular structure determination by mass and NMR spectroscopy; 6-23 nm: lattice parameters of lyotropic phases determined by small angle x-ray scattering (SAXS); 15-200 nm: schematic drawing of the LC phase created with Mathematica (Wolfram Research Inc.); 0.1-50 µm: determination by confocal microscopy, polarization microscopy and static light scattering (SLS); and 5-50 mm: visual inspection.

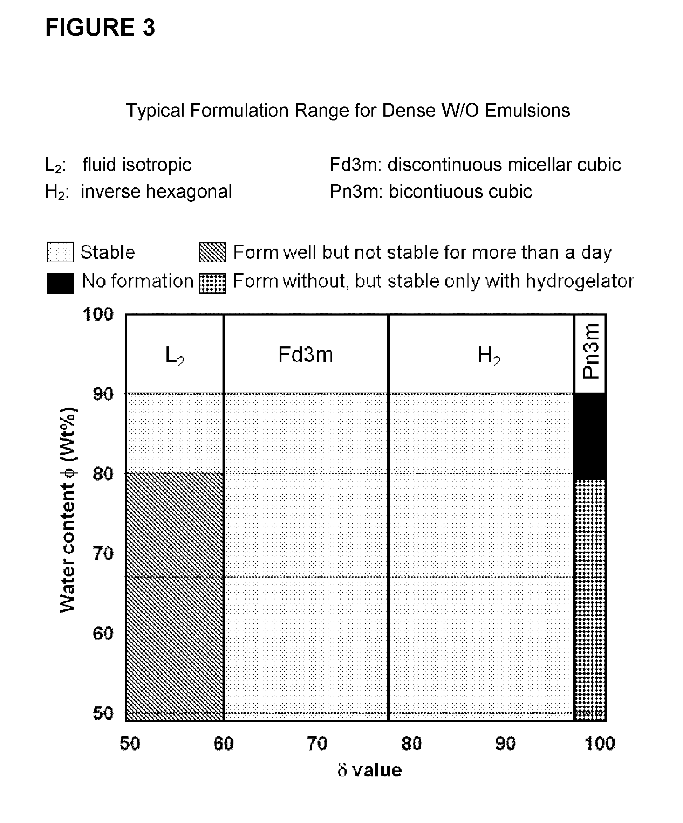

**FIGURE 3****: Formulation and stability range of water-in-oil emulsions according to the invention.** Different shadings in the diagram indicate the formation and stability ranges as indicated in the top legend. Stable nanostructured emulsions based on discrete micellar cubic (Fd3m) and hexagonal (H2) crystalline phases were produced at water contents between 50% and 90%, micellar cubic phases at a liquid-to-oil weight ratio between 60% and 78%, hexagonal phases at a liquid-to-oil weight ratio between 78% and 96%. Stable formulations (water-in-oil emulsions) could be produced within the dotted ranges. Fluid isotropic (L2) based emulsions could be produced for all water contents and at a liquid-to-oil weight ratio between 50% and 60%. However, they were stable only above 80% water content. Below 80% they were not stable (coalescence and sedimentation occurred within one day; shaded with tilted lines). The nanostructured emulsions based on bicontinuous cubic crystalline phases (Pn3m) could be produced up to 90% water content and at a liquid-to-oil weight ratio above 96%, but water drains out via bicontinuous water channels as time passes. The addition of a hydrogelator to the hydrophilic phase stabilized these nanostructured emulsions against water drainage (shaded with crossed lines). The Pn3m based emulsions could not be formed within the black range. This is most probably because of the increased amount of water resulting in a significantly lower viscosity than in the Pn3m cubic phase. The water content for nanostructured emulsions could be increased to values higher than 90% by pre-saturating the hydrophobic phase with water until its phase separation line (excess water boundary).

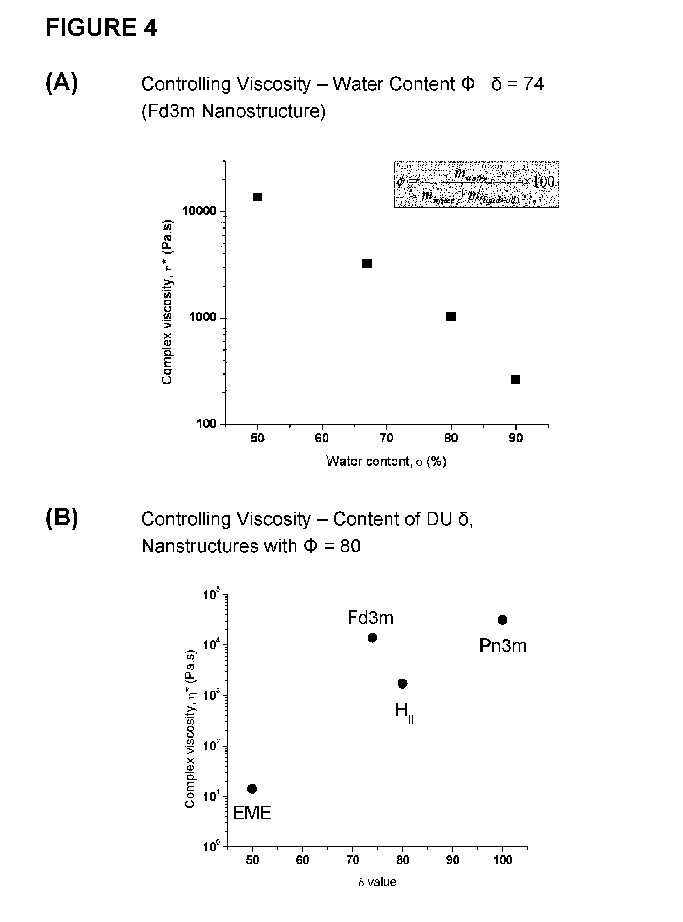

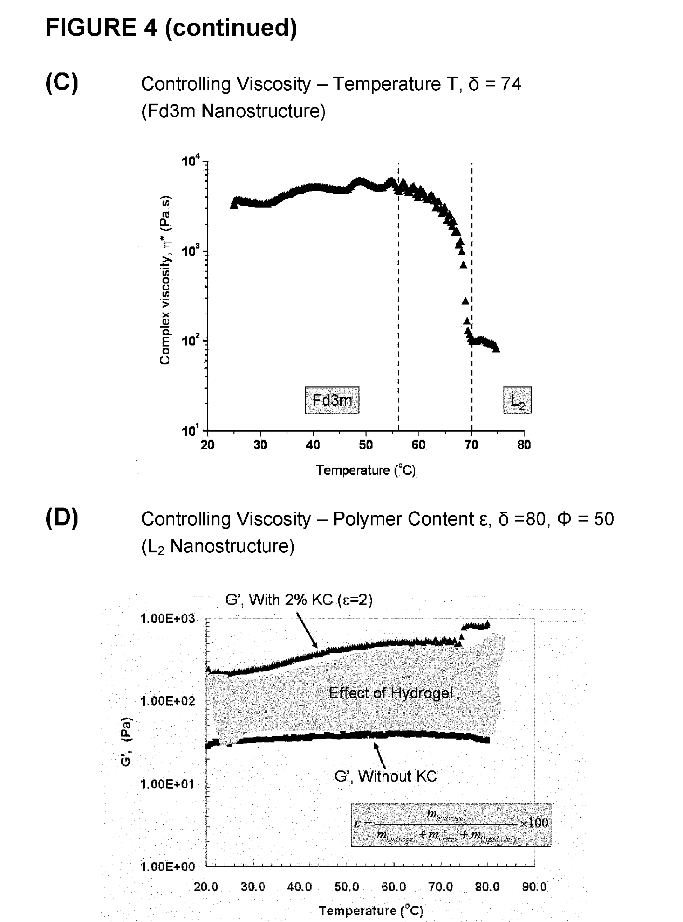

**FIGURE 4****: Control of the crystalline phase (complex viscosity, η*) of the lyotropic liquid crystalline nanostructures formed.** Control of the complex viscosity (η*) can be accomplished by varying one or more of the following parameters: water content (Φ) of the emulsion; the amount of oil, where (δ) represents the weight ratio of lipids to oils in the hydrophobic phase; the temperature (T); and the amount of a hydrogelator present (ε). All these rheological data were obtained from amplitude (stress) sweep measurements at a constant frequency of 1 Hz, at 25°C or, for analyzing reaction temperature, from 25-75°C over 3 h.

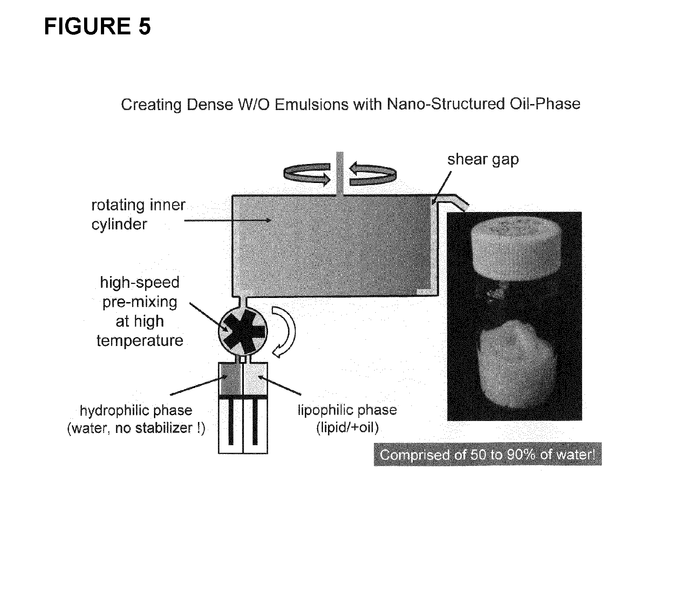

**FIGURE 5****: Preparation of a water-in-oil emulsion according to the invention.** Shown is an exemplary embodiment of a method according to the invention for the preparation of a water-in-oil emulsion via shearing: the hydrophilic and lipophilic (hydrophobic) phases are premixed with a high speed propeller and at high temperature to form raw emulsion which is then sheared the narrow gap (100 µm) of a Couette laminar flow shear device. Finally, the emulsion is cooled to ambient temperature and collected. The whole set up is thermally controlled.

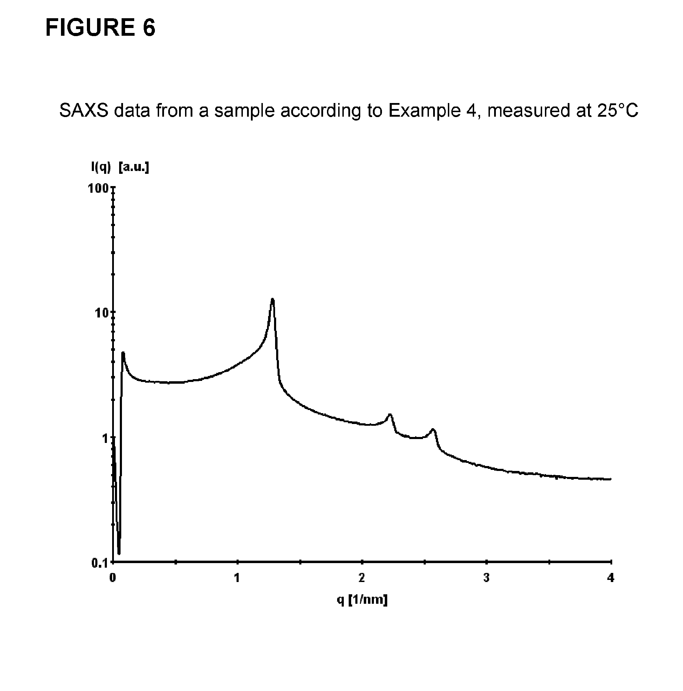

**FIGURE 6****: SAXS data from a sample according to Example 4, measured at 25°C.** The emulsion according to Example 4 was filled in a 1 mm vacuum tight capillary and placed in the temperature controlled sample holder of a SAXSess small-angle X-ray scattering camera (Anton Paar, Graz, Austria). This camera uses monochromatic X-rays, collimated by a Göbel mirror and defined by a block-collimator. The 2D pattern recorded with a CCD detector is vertically integrated into the scattering curve I(q) shown without further treatment. The data give a clear indication of the hexagonal structure.

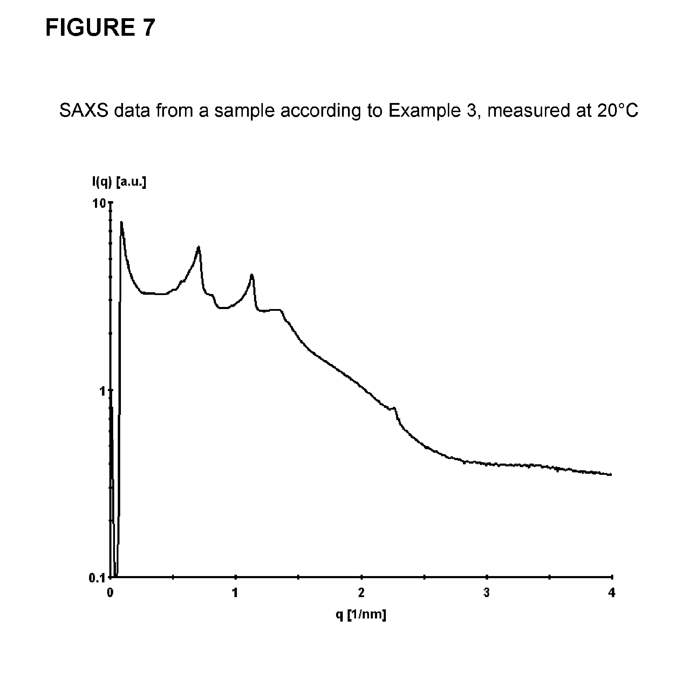

**FIGURE 7****: SAXS data from a sample according to Example 3, measured at 20°C.** The emulsion according to Example 3 was filled in a 1 mm vacuum tight capillary and placed in the temperature controlled sample holder of a SAXSess small-angle X-ray scattering camera (Anton Paar, Graz, Austria). This camera uses monochromatic X-rays, collimated by a Göbel mirror and defined by a block-collimator. The 2D pattern recorded with a CCD detector is vertically integrated into the scattering curve I(q) shown without further treatment. The data give a clear indication of the Fd3m structure.

### DETAILED DESCRIPTION OF THE INVENTION

The present invention is based on the unexpected finding that highly concentrated stable water-in-oil emulsions can be formulated without the use of an emulsion stabilizer for the water-oil-interface (neither molecular nor colloidal). This is accomplished by the assembly of lytropic liquid crystalline nanostructures that form the continuous hydrophobic phase of the emulsion. The emulsions described herein can be efficiently prepared by the application of shear forces at elevated temperatures. By controlling the experimental conditions during preparation, it is possible to prepare a "customized" emulsion having physicochemical properties that are adapted to a particular application of interest.

The present invention illustratively described in the following may suitably be practiced in the absence of any element or elements, limitation or limitations, not specifically disclosed herein.

Where the term "comprising" is used in the description and the claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun, e.g., "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like, in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of terms will be given in the following in the context of which the terms are used. The following terms or definitions are provided solely to aid in the understanding of the invention. These definitions should not be construed to have a scope less than understood by a person of ordinary skill in the art.

In a first aspect, the present invention relates to a water-in-oil emulsion, comprising a continuous hydrophobic phase in which the hydrophilic phase is dispersed, wherein:
(i) the emulsion has a total water content in the range of 30% to 95% (w/w);
(ii) the emulsion does not comprise an emulsion stabilizer;
(iii) the hydrophobic phase has a ratio of lipids to oils in the range of 50% to 96% (w/w); and
(iv) the continuous hydrophobic phase is made of lyotropic liquid crystalline nanostructures including 5% to 40% (w/w) water.

The term "emulsion", as used herein, denotes a mixture of two or more liquids that are normally immiscible (i.e. not mixable). In an emulsion, one liquid (the dispersed phase) is dispersed in the other (the continuous phase). The present invention is directed to water-in oil emulsions comprising a continuous hydrophobic (i.e. lipophilic) phase in which the hydrophilic phase is dispersed. Typically, the dispersed phase is statistically distributed. An emulsion according to the present invention differs from a common emulsion known in the art in a tow-fold manner: (i) it comprises a well-defined internal structure, that is, the continuous hydrophobic phase is made of lyotropic viscous liquid crystalline nanostructures including (i.e. inside) 5% to 40% (w/w) water, whereas common emulsions of the art typically do not exhibit a well-defined internal structure; and (ii) it does not comprise an emulsion stabilizer (also referred to as emulsifier), that is, a substance that stabilizes an emulsion. Examples of such emulsifiers include *inter alia* surface-active substances (surfactants; e.g., Pluronic® F127 or Tetronic® 150R1), detergents (e.g, Tween® 20), proteins (e.g., β-lactoglobulin or β-casein), and colloidal particles (including nanoparticles, such as silica or clay).

The term "liquid crystal", as used herein, refers to a state of a material or matter that exhibits properties between those of a conventional liquid and those of a solid crystal. For instance, a liquid crystal may flow like a liquid, but its molecules may be oriented in a crystal-like way. As used herein, a liquid crystal is referred to as "lyotropic" if it comprises two or more components that exhibit liquid-crystalline properties in certain concentration ranges, thereby inducing the formation of different phases. Such phases are made of amphiphilic molecules that have both hydrophilic and hydrophobic parts (i.e. polar and non-polar parts). Amphiphilic molecules self-assemble when mixed with water or other fluids, wherein the composition (e.g., the water content), the molecular characteristics of the molecules employed, their physicochemical natures, and the like changes the type of the self-assembled structures (reviewed, for example, in Seddon, J.M. and Templer, R.H. (1993) Philos. Trans. R. Soc. London 344, 377-401). Each of these different types has a different extent of molecular ordering.

For example, at low water content and/or low temperature conditions, lipid molecules stay either in amorphous state (herein referred to as "fluid isotropic" or "L₂" phase) or in a lamellar phase having crystalline (L_{γ}), gel (L_{β}) or liquid crystalline (L_{α}) polymorphic states (herein also referred to as "nanostructures"). With increasing hydration, two or three-dimensional phases of a state of higher order are formed, depending on the molecular shape. Long cylinders of either hydrocarbon chains or hydrophilic headgroups and water assemble to form two-dimensional columnar hexagonal phases (herein commonly referred to as "hexagonal" or "H" phase), which may have normal (type H1) or inverse (type H2) structural form. Three-dimensional phases include *inter alia* bicontinuous cubic phases (also referred to as "Im3m", "Pn3m", and "la3d" phases) and a micellar cubic phase (herein also referred to as "Fd3m" phase). All these crystalline phases are well known in the art (reviewed, e.g., Seddon, J.M. and Templer, R.H. (1995) in Handbook of Biological Physics (Lipowsky, R. and Sackmann, E., eds.), Elsevier Science B.V., Vol. 1, pp. 97-160). Various methods for analyzing nanostructures formed by such crystalline phases are well established in the art, such as small angle X-ray scattering, optical polarization microscopy, static light scattering, and rheological measurements.

In preferred embodiments, lyotropic liquid crystalline nanostructures of the hydrophobic phase have a crystalline phase being selected from the group consisting of fluid isotropic, hexagonal, bicontinuous cubic, and (discrete) micellar cubic, with the latter three being particularly preferred.

The continuous hydrophobic phase of an emulsion according to the present invention may comprise lyotropic liquid crystalline nanostructures of a single type, for example only having a hexagonal crystalline phase, only a discrete micellar cubic phase, or only a bicontinuous cubic phase. However, it may also be possible that combinations of two or more different crystalline phases are present, for example, (fluid isotropic and discrete micellar cubic), (lamellar and discrete micellar cubic), (discrete micellar cubic and hexagonal), or (hexagonal and bicontinuous cubic). Furthermore, it may be possible that two or more different forms of one crystalline phase are present, for example, (Im3m and Pn3m) bicontinuous cubic phases.

A water-in-oil emulsion of the present invention has a total water content being in the range of 30% to 95% (w/w; weight per weight, that is, the mass of water relative to the total mass of the emulsion). In some preferred embodiments, the total water content of the emulsion is at least 70% (w/w), and particularly preferably at least 75% (w/w). Typically, the emulsions have a water content being in the range of 40% to 90% (w/w) or of 50% to 80% (w/w), for example 40%, 42%, 45%, 48%, 50%, 52%, 55%, 58%, 60%, 62%, 65%, 68%, 70%, 72%, 75%, 78%, 80%, 82%, 85%, 88%, and 90% (w/w). The skilled person is well aware of various standard methods for determining the water content of an emulsion, such as NMR spectroscopy, mass spectroscopy, evaporation analysis, and chemical methods (e.g., Karl-Fischer reagent method).

The hydrophilic phase may be dispersed in the continuous hydrophobic phase in any two- or three-dimensional form. In specific embodiments, the hydrophilic phase is dispersed in form of droplets. The droplets may have a diameter (size) in the range of 0.1 to 100 µm or of 0.1 to 50 µm. Typically, the droplets have a diameter in the range of 0.1 to 50 µm, such as *inter alia* 0.5 µm, 1 µm, 2 µm, 5 µm, 8 µm, 10 µm, 12 µm, 15 µm, 18 µm, 20 µm, 22 µm, 25 µm, 28 µm, 30 µm, 32 µm, 35 µm, 38 µm, 40 µm, 42 µm, 45 µm, 48 µm, and 50 µm. The droplets may be monodispersed, that is, they have the same or substantially the same size (e.g., 1 µm mean ± 10%), or polydispersed, that is, they have different sizes (e.g., 2 µm and 10 µm, each mean ± 10%). In some embodiments, at least a part of the droplets fuse to form larger structures that may have another shape than droplets (for example, tubular-like shapes). The size of the droplets may be analyzed by various standard techniques, for example, by static light scattering or microscopy.

A water-in-oil emulsion of the present invention has in its hydrophobic phase a ratio of lipids to oils in the range of 50% to 96% (w/w; weight per weight). Typically, the ratio of lipids to oils is in the range of 60% to 95% (w/w) or of 70% to 90% (w/w). Hence, in some embodiments, the hydrophobic phase is only composed of lipids. In other embodiments, the hydrophobic phase is composed of a 1:1 (w/w) mixture of lipids and oils. Exemplary ratios of lipids to oils include *inter alia* 52% 55%, 58%, 60%, 62%, 65%, 68%, 70%, 72%, 75%, 78%, 80%, 82%, 85%, 88%, 90%, 92%, and 95% (w/w).

The term "lipids", as used herein, generally refers to amphiphilic molecules, that is, molecules having both hydrophilic and hydrophobic parts that can self-assemble in water or another solvent to form structures such as vesicles or micelles. Examples of lipids include *inter alia* monogylcerides, diglycerides, triglycerides, polyglycerol esters, phytantriol (i.e., 3,7,11,15-tetramethylhexadecane-1,2,3-triol), fatty acids, and phospholipids. All these classes of compounds are well known in the art and commercially available. In some embodiments, the emulsion comprises only a single class of lipid compounds (including one or more different molecular species), for example, only monoglycerides. In other embodiments, the emulsion comprises two or more classes of lipid compounds (each including one or more different molecular species), e.g., (monoglycerides and diglycerides) or (monoglycerides and polyglycerol esters). Monoglycerides are a particularly preferred class of lipids. In some specific embodiments, monoglycerides constitute at least 50% or at least 70% (w/w) of the lipids present, for example, 55%, 60%, 65%, 70%, 75%, 80%, 85%, 90%, and 95% (w/w). The lipids may be saturated or unsaturated. In some embodiments, the portion of saturated lipids is less than 30% or less than 15%, for example 25%, 20%, 15%, 10%, and 5%. The lipids may be naturally occurring or chemically synthesized and may be used without further purification, after partial enrichment or after full purification.

The term "oil", as used herein, denotes a hydrophobic/amphiphilic substance that is liquid at ambient or elevated (i.e. up to 40°C) temperature but soluble in organic solvents. Hence, an oil can also be considered a specific form of a lipid. An emulsion of the present invention may comprise one or more different oils. The oils may be naturally occurring or chemically synthesized and may be used without further purification, after partial enrichment or after full purification.

In particularly preferred embodiments, the oils employed in the hydrophobic phase are natural oils. Examples of natural oils that can be employed herein include *inter alia* almond oil, avocado oil, canola oil, castor oil, coconut oil, flaxseed oil, jojoba oil, lemon oil, macadamia nut oil, neem oil, olive oil, orange oil, palm oil, peanut oil, safflower oil, sunflower oil, tea tree oil, evening primrose oil, and walnut oil. It may also be possible to use mixtures of two or more natural oils, in particular of natural oils selected from the group indicated above, or to use mixtures of one or more natural oils and one or more synthetic (or semisynthetic) oils including silicone oils. Preferred oils include *inter alia* almond oil, sunflower oil, and evening primrose oil.

In specifically preferred embodiments, the continuous hydrophobic phase of the emulsion is made (or substantially made) of lytropic liquid crystalline nanostructures having a discrete micellar cubic phase (in particular, an Fd3m phase). The term "substantially made of", as used herein, denotes that at least 80%, preferably at least 90% of the nanostructures have a discrete micellar phase. Typically, such emulsions have a water content in the range of 50% to 90% (w/w) and a lipid-to oil weight ratio in the range of 60% to 78% (w/w). For example, such an emulsion may have a water content of *inter alia* 50%, 55%, 60%, 65%, 70%, 75%, 80%, 85% or 90% (w/w) in combination with a lipid-to oil weight ratio of *inter alia* 60%, 62%, 64%, 66%, 68%, 70%, 72%, 74%, 76% or 78% (w/w).

In other specifically preferred embodiments, the continuous hydrophobic phase of the emulsion is made (or substantially made) of lytropic liquid crystalline nanostructures having a hexagonal phase (in particular, a H2 phase). The term "substantially made of", as used herein, denotes that at least 80%, preferably at least 90% of the nanostructures have a hexagonal phase. Typically, such emulsions have a water content in the range of 50% to 90% (w/w) and a lipid-to oil weight ratio in the range of 78% to 94% (w/w). For example, such an emulsion may have a water content of *inter alia* 50%, 55%, 60%, 65%, 70%, 75%, 80%, 85% or 90% (w/w) in combination with a lipid-to oil weight ratio of *inter alia* 78%, 80%, 82%, 84%, 86%, 88%, 90%, 92% or 94% (w/w).

In yet other specifically preferred embodiments, the continuous hydrophobic phase of the emulsion is made (or substantially made) of lytropic liquid crystalline nanostructures having a fluid isotropic phase (in particular, a L2 phase). The term "substantially made of", as used herein, denotes that at least 80%, preferably at least 90% of the nanostructures have a fluid isotropic phase. Typically, such emulsions have a water content in the range of 75% to 90% (w/w) and a lipid-to oil weight ratio in the range of 50% to 60% (w/w). For example, such an emulsion may have a water content of *inter alia* 75%, 78%, 80%, 82%, 85%, 88% or 90% (w/w) in combination with a lipid-to oil weight ratio of *inter alia* 50%, 52%, 54%, 56%, 58% or 60% (w/w).

In yet other specifically preferred embodiments, the continuous hydrophobic phase of the emulsion is made (or substantially made) of lytropic liquid crystalline nanostructures having a bicontinuous cubic phase (in particular, a Pn3m phase). The term "substantially made of", as used herein, denotes that at least 80%, preferably at least 90% of the nanostructures have a bicontinuous cubic phase. Typically, such emulsions have a water content in the range of 50% to 90% (w/w) and a lipid-to oil weight ratio of at least 92% (w/w). For example, such an emulsion may have a water content of *inter alia* 50%, 55%, 60%, 65%, 70%, 75%, 80%, 85% or 90% (w/w) in combination with a lipid-to oil weight ratio of *inter alia* 92%, 94% or 96% (w/w). In specific embodiments, the emulsions further comprise a hydrogelator added to the hydrophilic phase in order to stabilize the emulsion against water drainage out of the bicontinuous nanostructures.

In specific embodiments, the emulsion further comprises a moisturizer being included in the hydrophilic phase in a range of 5% to 40% (w/w). The term "moisturizer", as used herein, denotes any compounds that increase hydration of a formulation, for example by reducing evaporation. Exemplary moisturizers include *inter alia* cetyl alcohol, silicone-based ingredients (e.g., cyclomethicone), petrolatum, and ceramides. Numerous moisturizers are well known in the art and commercially available.

In further preferred embodiments, the hydrophilic phase of the emulsion further comprises a hydrogelator. The term "hydrogelator", as used herein, denotes any water-swellable (hydrophilic) gel-forming polymers that are capable of becoming cross-linked with each other. Exemplary polymers include polysaccharides or polyacrylic acid derivatives. The gel-forming polymers may be naturally occurring polymers, synthetic polymers or mixtures thereof. Typically, they have an average molecular weight of 1 to 50 kDa, preferably of 1 to 30 kDa. Dalton. Suitable exemplary gel-forming polymers include *inter alia* cellulose derivatives (e.g., methylcellulose, ethylcellulose, hydroxyethyl-cellulose, and carboxymethyl-cellulose), polyacrylic acid derivatives (e.g., polyacrylic acid, polymethylacrylate, and polyethylacrylate), gums (e.g., agar, glucomannan, arabic gum, sodium alginate, and tragacanth). Preferable gel-forming polymers are carrageenans (i.e. linear sulfated galactan polysaccharides that are extracted from red seaweeds), with κ-carrageenan, τ-carageennan, and λ-carageenan being particularly preferred. It may also be possible to use combinations of different hydrogelators, for example (methylcellulose and κ-carrageenan) or (hydroxyethyl cellulose and agar).

In a further aspect, the present invention relates to a method for preparing a water-in-oil emulsion comprising a continuous hydrophobic phase made of lyotropic liquid crystalline nanostructures as defined herein, the method comprising:
(a) pre-mixing of the hydrophilic phase and the hydrophobic phase at a rotation speed of at least 4.000 rpm and at a temperature of at least 40°C, thus forming a raw emulsion;
(b) positioning the raw emulsion in a shear device; and
(c) applying a shear rate of at least 4.500 s⁻¹; and cooling the emulsion to ambient temperature, thus forming a continuous hydrophobic phase of the emulsion that is made of lyotropic liquid crystalline nanostructures.

In general, the emulsions according to the present invention are prepared at a temperature above the melting temperature of the corresponding lyotropic phase, that is, at a temperature where the hydrophobic phase is in liquid state. Thus, the reaction temperature for performing the steps of the above methods is at least 40°C, for example 45°C, 50°C, 55°C, 60°C, 65°C, 70°C, 75°C, 80°C, 85°C or 90°C. Typically, the preparation temperature is in the range between 50°C and 80°C. In respective specific embodiments, the preparation temperature for preparing emulsions comprising fluid isotropic (L2) nanostructures is at least 50°C, the preparation temperature for preparing emulsions comprising (discrete) micellar cubic (Fd3m) nanostructures is at least 60°C, the preparation temperature for preparing emulsions comprising hexagonal (H2) nanostructures is at least 78°C, and the preparation temperature for preparing emulsions comprising bicontinuous cubic (Pn3m) nanostructures is at least 95°C.

Typically, the method for producing the emulsions comprises the application of shear forces. However, in some alternative embodiments other techniques for preparing emulsions are applied, such as *inter alia* ultrasonication, homogenization, and microfluidization, all of which are well known in the art.

The shear device employed within the present invention may have any geometry, such as *inter alia* elongational flow and extrusion, Couette shear between sliding plates, concentric cylinders, cone and plate systems and parallel disks, and Poiseuille surface shear, all well established in the art (reviewed, e.g., in Baker, S.M. et al. (1994) Rev. Sci. Instrum. 65, 412-416; Kisilak, M. et al. (2001) Rev. Sci. Instrum. 72, 4305-4307).

In preferred embodiments, the shear device employed is a Couette laminar flow shear device. An exemplary device of this geometry that may be employed herein is described in Salenting et al. (Salentinig, S. et al. (2008) *J. Colloid Interface Sci.* **326**, 211-220).

Initially, a raw emulsion is prepared by premixing the hydrophilic phase (i.e. water or other solvents) and the hydrophobic phase (i.e. lipid or lipid + oil) in a suitable reaction chamber which may be directly attached to or being an integral part of the shear device. Any mechanical means allowing the mixing of the two phases may be employed. For example, a propeller type rotation device may be used. Such device may have a propeller made of three blades placed to each other at angles of 120°, a propeller made of four blades placed to each other at angles of 90° or a propeller made of five blades placed to each other at angles of 72°. The propeller may typically have a diameter in the range of 5 mm to 50 mm (e.g., 5 mm. 8 mm, 10 mm, 12 mm, 15 mm, 18 mm, 20 mm, 25 mm, 30 mm, 35 mm, 40 mm, 45 mm or 50 mm) and may typically be used with a rotation speed of at least 4.000 rpm (rounds per minute). For example, the pre-mixing step may be performed at a rotation speed of at least 5000 rpm, at least 8.000 rpm, at least 10.000 rpm, at least 12.000 rpm, at least 15.000 rpm, at least 18.000 rpm, at least 20.000 rpm, or even higher values (commonly the values given above are mean ± 200 rpm). The pre-mixing step is performed at a reaction temperature of at least 40°C (cf. above). In specific embodiments, the pre-mixing step is performed at a temperature of at least 60°C.

The resulting raw emulsion is then positioned in the shear device. For example, if the pre-mixing device is attached to or being an integral part of the shear device, the raw emulsion may be pressed (e.g., from the bottom) to the reaction cell of the shear device, preferably a Couette cell. Such Couette cell may have a shear gap between the rotor and the stator in the range of 50 µm to 1000 µm (= 1 mm), for example 100 µm, 200 µm or 500 µm. Typically, the rotating cylinder of the device has a diameter in the range between 20 mm and 100 mm, such as 20 mm, 30 mm, 40 mm, 50 mm, 60 mm, 70 mm, 80 mm or 90 mm. A shear rate of at least 4.500 s⁻¹ is applied (e.g., 5.000 s⁻¹, 8.000 s⁻¹, 10.000 s⁻¹, 12.000 s⁻¹, 15.000 s⁻¹, 18.000 s⁻¹, 20.000 s⁻¹, 22.000 s⁻¹, 25.000 s⁻¹, 28.000 s-¹, 30.000 s-¹, 32.000 s⁻¹, 35.000 s⁻¹, 38.000 s⁻¹, 40.000 s⁻¹, 42.000 s⁻¹, 45.000 s⁻¹ or even higher values (commonly the values given above are mean ± 200). When using a Coulette cell having a shear gap of 100 µm, a shear rate of 25.000 s⁻¹ corresponds to an angular speed of the rotating cylinder (having a diameter of 60 mm) of about 800 rpm. Typically, the angular speed of such rotating cylinder may be varied from 800 rpm to 2500 rpm, resulting in a corresponding shear rate in the range from 25.000 to 78.500 s⁻¹. In preferred embodiments, a shear rate of at least 25.000 s⁻¹ is applied. In further preferred embodiments, the pre-mixing step of the method is performed at a rotation speed of at least 20.000 rpm, and a shear rate of at least 25.000 s⁻¹ or at least 35.000 s⁻¹ is applied.

Once the hydrophilic and hydrophobic phases are mixed under high pre-shear (about 20 000 rpm), the nanostructured emulsions, as described herein, are assumed to assemble via topological reorganization: the raw emulsion undergoes mechanical rupture by means of shear-induced elongation in the Couette cell. A continuous hydrophobic film of lyotropic liquid crystalline nanostructures is then formed, which, when cooled down to ambient temperature (e.g., in the range of 15°C to 25°C), encapsulates large amounts of water (≥ 50% w/w) while organizing into the nanostructured emulsion. This hypothesis is supported by two observations: (i) the fact that the nanostructured emulsions is not produced when the pre-shear step was omitted; and (ii) the viscosity of the resulting nanostructured emulsion changes with varying lyotropic nanostructures. Thus, the liquid crystalline phases of interest determine the preparation (instrumental conditions) and formation (topological transformations) of hierarchically ordered nanostructured emulsions under excess water conditions.

The method described herein may be performed as a "batch" process or as a "continuous" process.

In specific embodiments, the method further comprises controlling the crystalline phase of the lyotropic liquid crystalline nanostructures formed in the hydrophobic phase by adjusting any one or more of the preparation parameters selected from the group consisting of preparation temperature, total water content of the emulsion, ratio of lipids to oils in the hydrophobic phase of the emulsion, and presence of a hydrogelator in the hydrophilic phase. In particular, any one or more of these parameters are monitored (and adjusted accordingly, if applicable) in order to produce a water-in-oil emulsion having desired nanostuctures (e.g., lyotropic liquid crystalline nanostructures having a hexagonal phase). Thus, by modifying the preparation parameters it is possible to prepare "customized" or "tailored" emulsions that are specifically adapted for a particular application. Exemplary preparation parameters resulting in lyotropic liquid crystalline nanostructures of various types have been described above.

In another aspect, the present invention relates to the use of a water-in-oil emulsion, as defined herein, for the preparation of a cosmetic or pharmaceutical composition, the composition comprising one or more active ingredients. In other words, the emulsions are employed as delivery systems for the one or more active ingredients, such as vitamins, plant extracts, pharmaceuticals, and the like. In specific embodiments, the water-in-oil emulsions, as defined herein, is used for the preparation of food composition.

In preferred embodiments, the cosmetic or pharmaceutical composition prepared is for topical application, that is, for the administration on body surface, such as the skin or mucous membranes. Examples of such topical compositions or dosage forms include *inter alia* solutions, suspensions, dispersions, tinctures, gels, topical sprays, topical foams, gels, ointments, creams, lotions, mousses, and balms, with the latter four being preferred. Creams, lotions, mousses, and balms primarily differ with regard to their respective viscosities. A cream is a semi-solid emulsion, that is, it has a medium viscosity. In contrast, a lotion is a low- to medium-viscosity preparation intended for application to unbroken skin. A mousse is typically a foam-like formulation. Finally, a balm (also referred to as liniment) has a similar viscosity as a lotion (i.e. being significantly less viscous than a cream) but unlike a lotion a balm is applied with friction, that is, a liniment is always rubbed in.

All these topical compositions or dosage forms as well as methods for their preparation are well established in the art (see, e.g., with respect to pharmaceutical compositions: Niedner, R., and Ziegenmeyer, J. (1997) Dermatika. Therapeutischer Einsatz, Pharmakologie und Pharmazie. Wissenschaftliche Verlagsgesellschaft mbH, Stuttgart, Germany; Gennaro, A.L. and Gennaro, A.R. (2000) Remington: The Science and Practice of Pharmacy, 20th Ed., Lippincott Williams & Wilkins, Philadelphia, PA; Niazi, S.K. (2004) Handbook of Pharmaceutical Manufacturing Formulations, CRC Press, Boca Raton, FL).

The invention is further described by the figures and the following examples, which are solely for the purpose of illustrating specific embodiments of this invention, and are not to be construed as limiting the scope of the invention in any way.

### EXAMPLES

### Example 1: Formation of a 50% water-in-oil emulsion based on a micellar-cubic lipid phase

16 g "oil phase" (hydrophobic phase) were prepared by mixing at about 30°C. This oil phase consisted of 4.48 g glycerol monoolein (GMO), 6.72 g diglycerol monoolein (DGMO), 4.67 g almond oil, and 0.13 g tee-tree oil.

This oil phase was then mixed with the water phase, containing 1.2% κ-carrageenan, in a volume ratio of 1:1 in the premixing chamber of a shear device at 20.000 rpm and sheared with a shear rate of 31.410 s⁻¹ at a constant temperature of 80°C. The resulting emulsion was immediately cooled to a temperature below 40°C in a flow-through cooler to ensure the formation of the micellar cubic phase.

### Example 2: Formation of a 67% water-in-oil emulsion based on a micellar-cubic lipid phase

11 g "oil phase" (hydrophobic phase) were prepared by mixing at about 30°C. This oil phase consisted of 3.08 g GMO, 4.62 g DGMO, 1.65 g almond oil, and 1.65 g sunflower oil.

This oilphase was then mixed with the water phase, containing 1.2% κ-carrageenan, in a volume ratio of 1:2 in the premixing chamber of a shear device at 20.000 rpm and sheared with a shear rate of 31.410 s⁻¹ at a constant temperature of 80°C. The resulting emulsion was immediately cooled to a temperature below 40°C in a flow-through cooler to ensure the formation of the micellar cubic phase.

### Example 3: Formation of a 50% water-in-oil emulsion based on a micellar-cubic lipid phase

160 g "oil phase" (hydrophobic phase) were prepared by mixing at about 30°C. This oil phase consisted of 44.8 g GMO, 67.2 g DGMO, and 48 g evening primerose oil.

This oil phase was then mixed with the water phase, containing 1.2% κ-carrageenan, in a volume ratio of 1:1 in the premixing chamber at 20.000 rpm and sheared with a shear rate of 31.410 s⁻¹ at a constant temperature of 90°C. The resulting emulsion was immediately cooled to a temperature below 40°C in a flow-through cooler to guarantee the formation of the micellar cubic phase (for SAXS data of this sample see Fig. 7).

### Example 4: Formation of a 50% water-in-oil emulsion based on a inverse hexagonal lipid phase

11 g "oil phase" (hydrophobic phase) were prepared by mixing at about 30°C. This oil phase consisted of 8.24 g MLO (Dimodan U/J ®), 0.92 g almond oil, 0.92 g triolein, and 0.92 g R(+)-limonene.

This oilphase was then mixed with the water phase, containing 1.2% κ-Carrageenan, in a volume ratio of 1:1 in the premixing chamber at 20.000 rpm and sheared with a shear rate of 47.120 s⁻¹ at a constant temperature of 90°C. The resulting emulsion was immediately cooled to a temperature below 40°C in a flow-through cooler to ensure the formation of the inverse hexagonal phase (for SAXS data of this sample see Fig. 6).

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Water-in-oil emulsion, comprising a continuous hydrophobic phase in which the hydrophilic phase is dispersed, wherein:
(i) the emulsion has a total water content in the range of 30% to 95% (w/w);
(ii) the emulsion does not comprise an emulsion stabilizer;
(iii) the hydrophobic phase has a ratio of lipids to oils in the range of 50% to 96% (w/w); and
(iv) the continuous hydrophobic phase is made of lyotropic liquid crystalline nanostructures including 5% to 40% (w/w) water.

2. The water in-oil emulsion of claim 1, wherein the lyotropic liquid crystalline nanostructures have a crystalline phase being selected from the group consisting of fluid isotropic, hexagonal, bicontinuous cubic, and discrete micellar cubic.

3. The water in-oil emulsion of claim 1 or 2, wherein the hydrophilic phase is dispersed in form of droplets having a diameter in the range of 0.1 to 50 µm.

4. The water in-oil emulsion of any one of claims 1 to 3, wherein the total water content is at least 70% (w/w), and particularly at least 75% (w/w).

5. The water in-oil emulsion of any one of claims 1 to 4, wherein the oils are natural oils.

6. The water in-oil emulsion of any one of claims 1 to 5, wherein the hydrophilic phase further comprises a hydrogelator.

7. Method for preparing a water-in-oil emulsion comprising a continuous hydrophobic phase made of lyotropic liquid crystalline nanostructures as defined in any one of claims 1 to 6, the method comprising:
(a) pre-mixing of the hydrophilic phase and the hydrophobic phase at a rotation speed of at least 4.000 rpm and at a temperature of at least 40°C, thus forming a raw emulsion;
(b) positioning the raw emulsion in a shear device; and
(c) applying a shear rate of at least 4.500 s⁻¹; and cooling the emulsion to ambient temperature, thus forming a continuous hydrophobic phase of the emulsion that is made of lyotropic liquid crystalline nanostructures.

8. The method of claim 7, wherein the shear device is a Couette laminar flow shear device.

9. The method of claim 7 or 8, further comprising:
controlling the crystalline phase of the lyotropic liquid crystalline nanostructures formed in the hydrophobic phase by adjusting any one or more of the preparation parameters selected from the group consisting of preparation temperature, total water content of the emulsion, ratio of lipids to oils in the hydrophobic phase of the emulsion, and presence of a hydrogelator in the hydrophilic phase.

10. The method of any one of claims 7 to 9, wherein the pre-mixing step is performed at a temperature of at least 60°C.

11. The method of any one of claims 7 to 10, wherein the pre-mixing step is performed at a rotation speed of at least 15.000 rpm and wherein a shear rate of at least 25.000 s⁻¹ is applied.

12. Use of a water-in-oil emulsion as defined in any one of claims 1 to 6 for the preparation of a cosmetic or pharmaceutical composition, the composition comprising one or more active ingredients.

13. The use of claim 12, wherein the cosmetic or pharmaceutical composition is for topical application, in particular being selected from the group consisting of creams, lotions, mousses, and balms.
